# EUROPEAN PATENT APPLICATION

(11) **EP 4 227 004 A1**
(43) Date of publication of application: **16.08.2023**
(21) Application number: 21877928.8
(22) Date of filing: 05.10.2021
(51) Int. Cl.: B01L 7/00, B01L 3/02, B01L 3/00, G01N 35/10, C12Q 1/686

(54) **NUCLEIC ACID AMPLIFICATION TEST APPARATUS, AND AUTOMATIC SAMPLE ANALYSIS SYSTEM HAVING SAME**

(30) Priority: 05.10.2020 KR 20200128151
(71) Applicant: Bioneer Corporation, Daejeon 34302 (KR)
(72) Inventor: PARK, Han Oh, Sejong-si 30151 (KR); PARK, Hanee, Daejeon 35251 (KR); LEE, Jin Il, Daejeon 34011 (KR); KIM, Jong Kab, Daejeon 34008 (KR); KWON, Eun Yeong, Daejeon 35231 (KR)
(74) Representative: ABG Intellectual Property Law, S.L.
(86) International application number: PCT/KR2021/013552
(87) International publication number: WO 2022/075676

(57) **Abstract**

The present invention relates to a nucleic acid amplification test apparatus, and an automatic sample analysis system having same and, more particularly, to a nucleic acid amplification test apparatus in which separation and purification, dispensation, amplification, and testing of samples are performed integrally, and an automatic sample analysis system having same. Disclosed in the present invention is a nucleic acid amplification test apparatus comprising: a housing (30) having an inner space (S1) isolated from the outside; a multi-well plate insertion unit (100) into which a multi-well plate (40) having a plurality of reaction tubes (1) accommodating a target nucleic acid solution is inserted through an automatic purification and dispensing apparatus (10); a sealing plate insertion unit (200) into which a sealing plate (50) having a sealing means for sealing the inlets of the plurality of reaction tubes (1); a fluorescence detection unit (400) disposed on an upper side of the sealing plate insertion unit (200) and detecting a target nucleic acid in the reaction tubes (1); and a temperature control block (500) disposed on a lower side of the multi-well plate insertion unit (100) and controlling the temperature of the reaction tubes (1), wherein the reaction tubes (1) are moved relatively so as to be adjacent to the sealing means to be then sealed by the sealing means.

## Description

### TECHNICAL FIELD

The present invention relates to a nucleic acid amplification test apparatus, and an automatic sample analysis system having the same and, more particularly, to a nucleic acid amplification test apparatus in which separation and purification, dispensation, amplification, and testing of samples are performed integrally, and an automatic sample analysis system having the same.

### BACKGROUND ART

A gene amplification test method is an in vitro diagnostic testing (IVD testing) technology that amplifies a gene having a specific sequence to determine whether the gene exists and is used in various fields such as food inspection, GMO inspection, as well as pathogenic microorganism inspection and genotype inspection of various animals and plants, including humans.

In order to perform an accurate gene amplification test from various biological samples, first, a nucleic acid extraction process of removing various reaction inhibitors contained in the sample, which inhibits the gene amplification reaction, from the sample to acquire highpurity target nucleic acid is required.

The target nucleic acid extracted in this manner is mixed with a gene amplification solution to perform the gene amplification reaction, and then, DNA corresponding to a length of DNA of a gene amplification product is confirmed, or fluorescence generated from the gene amplification product is confirmed to complete the gene amplification test.

More specifically, the gene amplification test may be a method for amplifying a target nucleic acid, and various methods such as PCR, nested PCR, RT/PCR, and isothermal nucleic acid amplification have been developed. Generally, the gene amplification test may be performed through a preparation process of mixing the extracted target nucleic acid with the gene amplification reaction solution to prepare a gene amplification reactant and a reaction process of proceeding the reaction.

In particular, the gene amplification reaction process only needs to maintain a constant temperature in the case of the isothermal amplification, but when using the PCR, a heating and cooling process is required for temperature cycling reaction. In this process, the temperature has to be raised, and thus, a reaction tube is usually sealed to prevent evaporation from occurring.

In spite of the release of various automation systems, in the related art, after the reactant containing the target nucleic acid is dispensed from an automatic purification and dispensing apparatus in order to seal an inlet, which is an accommodation portion of the reaction tube, a user directly seals the inlets of the plurality of reaction tubes by using a sealing means such as a sealing film or a sealing film and then puts the target nucleic acid into the automatic purification and dispensing apparatus.

In this process, since the sealing of the inlet of the reaction tube is performed through the user's direct intervention, a preparation time for the gene amplification reaction process increases, and an entire test time of the gene amplification test increases together. As a result, there is a problem in that, since the entire processes of the gene amplification test are not fully automated, inspection efficiency is deteriorated, and cost and time increase.

In addition, since the user has to directly seal the inlet of the reaction tube with the sealing film in a state in which the reactant in the reaction tube is exposed from the outside of the nucleic acid amplification test apparatus, there is a problem that the reactant in the reaction tube may be contaminated by aerosol or the like.

### DISCLOSURE OF THE INVENTION

### TECHNICAL PROBLEM

In order to solve the above problems, an object of the present invention is to provide a nucleic acid amplification test apparatus, in which a process of dispensing a solution into a reaction tube, a process of performing sealing, and a nucleic acid amplification test process are continuously and automatically performed, and an automatic sample analysis system having the same.

In addition, in order to solve the above problems, another object of the present invention is to provide a nucleic acid amplification test apparatus capable of automatically moving to dispense a reaction solution or nucleic acid sample solution into a multi-well plate, and an automatic sample analysis system having the same.

### TECHNICAL SOLUTION

The present invention is invented to achieve the objects as described above, and the present invention discloses a nucleic acid amplification test apparatus of an automatic sample analysis system which includes an automatic purification and dispensing apparatus (10) configured to purify and dispense a target nucleic acid that is an analysis target, and a nucleic acid amplification test apparatus (20) configured to amplify and measure acquired from the automatic purification and dispensing apparatus (10), the nucleic acid amplification test apparatus including: a housing (30) having an inner space (S1) that is isolated from the outside; a multi-well plate insertion unit (100) in which a multi-well plate (40) provided with a plurality of reaction tubes (1), in which a target nucleic acid solution is accommodated, is inserted through an automatic purification and dispensing apparatus (10); a sealing plate insertion unit (200) into which a sealing plate (50) provided with a sealing means for sealing inlets of the plurality of reaction tubes (1); a fluorescence detection unit (400) disposed above the sealing plate insertion unit (200) to detect the target nucleic acid in the reaction tubes (1); and a temperature control block (500) disposed below the multi-well plate insertion unit (100) to control a temperature of each of the reaction tubes (1), wherein the reaction tubes (1) relatively move to be adjacent to the sealing means so as to be sealed by the sealing means.

The temperature control block (500) may allow the reaction tubes (1) to ascend to be in closely contact with the sealing unit through elevation driving so that the reaction tubes (1) are sealed by the sealing means.

The temperature control block (500) may include: a heating block (510) including a thermoelectric module (520) and disposed below the multi-well plate insertion unit (100) to control the temperature of the reaction tube (1) by exchanging heat with the reaction tube (1); a heat dissipation unit (800) provided below the heating block (510) to discharge the heat generated in the thermoelectric module (520) to the outside; an elevation driving unit (600) configured to drive the heat dissipation unit (800) vertically to allow the heating block (510) configured support the multi-well plate (40) to move vertically so as to drive a position of the multi-well plate (40) vertically.

The heating block (510) may include the thermoelectric module (520) and a reaction block (530) having a bottom surface that is in a surface contact with the thermoelectric module (520) and a top surface, in which a plurality of insertion grooves (531) into which the plurality of reaction tubes (1) are inserted are formed.

The heating block (510) may further include a reaction cover (540) coupled to the reaction block (530) so as to surround an outer surface of the reaction block (530).

The heating block (510) may include a measurement sensor (550) configured to sense the sealing plate (50) disposed thereabove so as to identify whether the sealing plate (50) exists.

The heat dissipation unit (800) may include: a heat dissipation plate (811) provided to be in surface contact with a lower side of the thermoelectric module (520) and a plurality of heat dissipation fins (812) provided on the heat dissipation plate (811); and a plurality of side surface parts (820) and bottom surface parts (830) coupled to the heat dissipation plate (811) so that the plurality of heat dissipation fins (812) are disposed in a separation space (S2) that is separated from the inner surface (S1) to form the separation space (S2) together with the heat dissipation plate (811).

The heat dissipation unit (800) may include an air circulation part (840) provided to independently communicate with the separation space (S2) and the outside of the housing (30) so that internal air of the separation space (S2) is circulated with external air of the housing (30).

The air circulation part (840) may include: a plurality of connection tubes (841) connected between the bottom surface part (830) and the housing (30); and an air circulation fan (842) installed in at least one of the plurality of connection tubes (841) to suction air outside the housing (30) or discharge air inside the separation space (S2) .

The elevation driving unit (600) may elevate the heat dissipation unit (800) to allow the multi-well plate (40) to move so that the plurality of reaction tubes (1) are attached to the sealing means.

The multi-well plate insertion unit (100) may include: a support part (110) configured to support an edge of the multi-well plate (40) in which the plurality of reaction tubes (1) are provided; and a driving part (120) configured to drive the support part (110) between a dispensing position (P) of the automatic purification and dispensing apparatus (10) and an amplification position in the housing (30).

The housing (30) may be provided with a shutter (60) configured to carry in and out of the multi-well plate insertion unit (100).

The sealing means may include a sealing film (51) or a sealing cap (52) configured to seal the inlet of the reaction tube (1).

The nucleic acid amplification test apparatus may further include an automatic sealing unit (300) configured to press the sealing means toward the multi-well plate (40) so that the sealing means is sealed to the inlet of the reaction tube (1), wherein the automatic sealing unit (300) may include a pressing part (310) provided to be movable above the sealing plate insertion unit (200) to directly or indirectly press the sealing means toward the multi-well plate (40).

The temperature control block (500) may drive and support the reaction tubes (1) to be elevated so that the plurality of reaction tubes (1) and the sealing means are in close contact with each other.

The pressing part (310) may include: a pressing roller (311) configured to seal the sealing means to the inlet of the reaction tube (1) through horizontal movement rolling in the state of directly or indirectly pressing the sealing means at the upper side of the sealing plate insertion unit (200); and a roller driving part (900) configured to drive the pressing roller (311).

The automatic sealing unit (300) may further include a heating part (320) provided between the pressing part (310) and the sealing plate seated on the sealing plate insertion unit (200) to create high-temperature environments at an inlet-side of the reaction tube (1) of the multi-well plate (40) .

The heating part (320) may have elasticity so that pressing force through the pressing part (310) is transmitted to the sealing means.

The heating part (320) may have through-holes (321) corresponding to the plurality of reaction tubes (1) to detect the target nucleic acid through the fluorescence detection unit (400) disposed above the sealing plate insertion unit (200) with respect to the reaction tubes (1).

The fluorescence detection unit (400) may include: a detection part (430) configured to irradiate excitation light with which the target nucleic acid reacts toward the reaction tube (1) and sense fluorescence according to the reaction of the target nucleic acid; and a detection driving part (440) configured to drive the detection part (430) to be movable above the sealing plate insertion unit (200).

The detection part (430) may include: a light source part (410) configured to irradiate the excitation light, which the target nucleic acid reacts, toward the reaction tube (1); and a detection sensor (420) configured to sense the fluorescence according to the reaction of the target nucleic acid through the irradiated excitation light.

The nucleic acid amplification test apparatus may further include a scan unit (700) configured to scan an identification information code attached to the reaction tube (1) so as to acquire target nucleic acid information.

The scan unit (700) may scan a position information code attached to the sealing plate (50) to identify whether the sealing means exists.

In addition, the present invention discloses an automatic sample analysis system including: an automatic purification and dispensing apparatus (10) configured to purify and dispense a target nucleic acid to dispense the target nucleic acid into a plurality of reaction tubes (1) at a dispensing position (P); and a nucleic acid amplification test apparatus (20) configured to measure a target nucleic acid solution, which is received from the automatic purification and dispensing apparatus (10) and accommodated in the plurality of reaction tubes (1), in real-time, wherein the nucleic acid amplification test apparatus (20) includes: a housing (30) having an inner space (S1); and a multi-well plate insertion unit (100) configured to support a multi-well plate (40) provided with a plurality of reaction tubes (1) and to be installed to be movable between the dispensing position (P) and the inner space (S1).

The multi-well plate insertion unit (100) may include: a support part (110) configured to support an edge of the multi-well plate (40) in which the plurality of reaction tubes (1) are provided; and a driving part (120) configured to drive the support part (110) between a dispensing position ()) of the automatic purification and dispensing apparatus (10) and an amplification position, at which amplification of the target nucleic acid is performed, in the housing (30).

The housing (30) may be provided with a shutter (60) configured to carry in and out of the multi-well plate insertion unit (100).

### ADVANTAGEOUS EFFECTS

The nucleic acid amplification test apparatus and the automatic sample analysis system having the same according to the present invention may have the advantages in which, since the multi-well plate is driven to be reciprocated between the nucleic acid amplification test apparatus and the automatic purification and dispensing apparatus, the real-time PCR through the dispensing to the multi-well plate and the sealing process is automatically performed automatically perform the entire processes of the target nucleic acid solution dispensing and the nucleic acid amplification test from the automatic nucleic acid purification unit, thereby immediately acquiring the result after the apparatus operates without the intermediate work of the inspector.

The nucleic acid amplification test apparatus and the automatic sample analysis system according to the present invention may have the advantages in which the inlet of the reaction tube is automatically sealed to improve the sealing rate and reduce the overall detection time of the target nucleic acid, thereby improving the detection efficiency.

In particular, the nucleic acid amplification test apparatus and the automatic sample analysis system having the same according to the present invention may have the advantages in which the inlet of the reaction tube is automatically sealed to enable the constant sealing and simplify the sealing mechanism compared to the conventional method in which the user directly performs the sealing.

In addition, the nucleic acid amplification test apparatus and the automatic sample analysis system having the same according to the present invention may have the advantages in which the inlet of the reaction tube is automatically sealed to minimize the sample contamination problem such as the mixing of the foreign substances in the reaction tube compared to the conventional method in which the user directly performs the sealing, thereby improving the accuracy in detection.

In particular, the nucleic acid amplification test apparatus and the automatic sample analysis system having the same according to the present invention may have the advantages in which the sealing space, in which the inlet of the reaction tube is sealed, is isolated from the outside to minimize the sample contamination problem such as the mixing of the foreign substances in the reaction tube while waiting for dispensing and sealing in the process of performing the purification of the nucleic acid, thereby improving the accuracy in detection.

### BRIEF DESCRIPTION OF THE DRAWINGS

FIG. 1 is a perspective view illustrating a schematic configuration of an automatic sample analysis system according to the present invention.
FIG. 2 is a front view illustrating the schematic configuration of the automatic sample analysis system of FIG. 1.
FIG. 3 is a view illustrating a driving state of a nucleic acid amplification test apparatus in the automatic sample analysis system of FIG. 1.
FIG. 4 is a plan view illustrating a configuration of an automatic sealing unit of the nucleic acid amplification test apparatus of FIG. 1.
FIG. 5 is a cross-sectional view illustrating a configuration of the automatic sealing unit of the nucleic acid amplification test apparatus of FIG. 1.
FIGS. 6a to 6c are cross-sectional views illustrating a state in which a multi-well plate of the nucleic acid amplification test apparatus of FIG. 1 is elevated, wherein FIG. 6a is a cross-sectional view illustrating a state in which the multi-well plate is inserted into a housing, FIG. 6b is a cross-sectional view illustrating a state in which a portion of the multi-well plate is elevated to support a reaction tube, and FIG. 6c is a cross-sectional view illustrating a state in which the multi-well plate is elevated so that the reaction tube and a sealing film are in contact with each other.
FIG. 7 is a cross-sectional view illustrating a configuration of a temperature control block of the nucleic acid amplification test apparatus of FIG. 1.
FIG. 8 is a cross-sectional view illustrating configurations of the temperature control block and an elevation driving unit of the nucleic acid amplification test apparatus of FIG. 1.

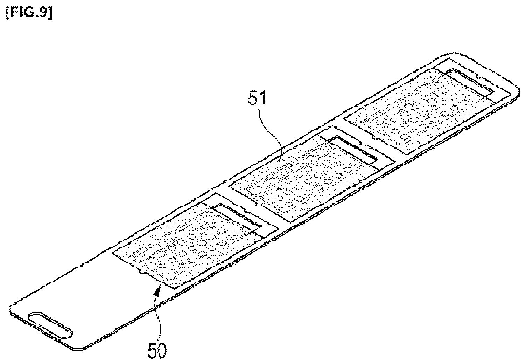

FIG. 9 is a perspective view illustrating a configuration of a sealing film-type sealing plate of the nucleic acid amplification test apparatus of FIG. 1.
FIG. 10 is a perspective view illustrating a configuration of a sealing cap-type sealing plate of the nucleic acid amplification test apparatus of FIG. 1.

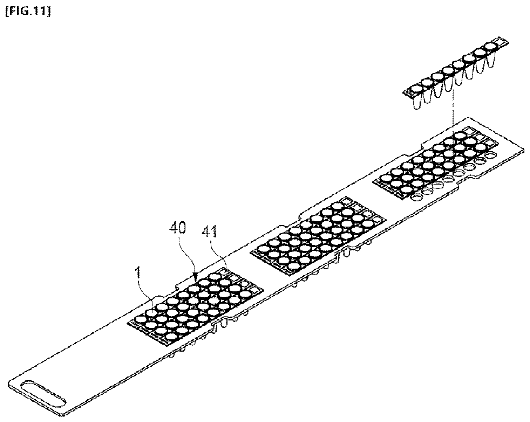

FIG. 11 is a perspective view illustrating a configuration of the multi-well plate on which a plurality of reaction tubes for nucleic acid amplification in the nucleic acid amplification test apparatus of FIG. 1.

### MODE FOR CARRYING OUT THE INVENTION

Hereinafter, a nucleic acid amplification test apparatus and an automatic sample analysis system having the same according to the present invention will be described with reference to the accompanying drawings.

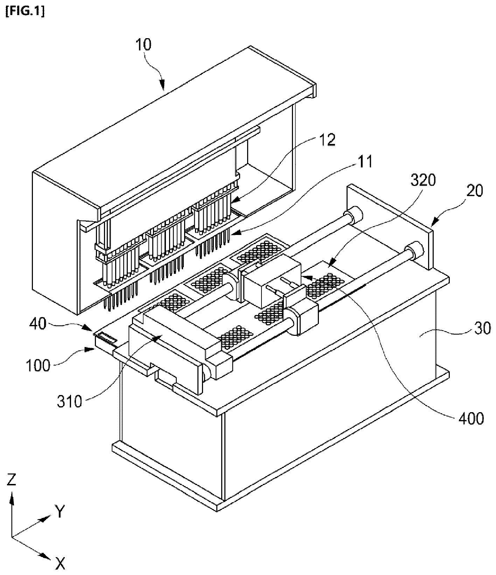

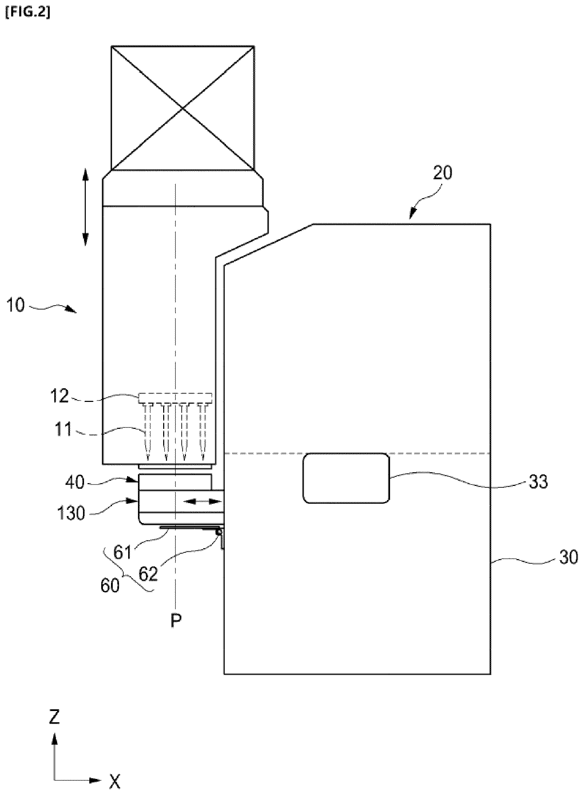

As illustrated in FIGS. 1 to 3, the automatic sample analysis system according to the present invention may be an automatic sample analysis system in which qualitative or quantitative analysis of a target nucleic acid is performed at the same time and may include: an automatic purification and dispensing apparatus 10 that separates and purifies the target nucleic acid to dispense the target nucleic acid into a plurality of reaction tubes 1 at a dispensing position P; and a nucleic acid amplification test apparatus 20 that measures the target nucleic acid, which is received from the automatic purification and dispensing apparatus 10, in the plurality of reaction tubes 1 in real-time.

Here, a sample to be analyzed has a configuration including the target nucleic acid to be inspected, and various configurations are possible.

For example, the sample may be a biological sample and may mean blood, urine, tissue, saliva, sputum, etc., containing the target nucleic acid obtained from living organisms, and may further include the living body itself such as animals, plants, and microorganisms.

Thus, the automatic sample analysis system according to the present invention may perform analysis by purifying the target nucleic acid to be analyzed from the sample and detecting the target nucleic acid through the amplification of the target nucleic acid.

The automatic purification and dispensing apparatus 10 may be a configuration that separates and purifies the target nucleic acid to dispense the target nucleic acid into the plurality of reaction tubes 1 at the dispensing position P, and various configurations are possible.

That is, if the automatic purification and dispensing apparatus 10 is configured to amplify the nucleic acid through the nucleic acid amplification test apparatus 20 to be described later and purify the target nucleic acid to be measured in real-time, thereby dispensing the target nucleic acid into the nucleic acid amplification test apparatus 20, the automatic purification and dispensing apparatus 10 may be applied to any type of apparatuses disclosed in the related art and thus will be briefly described below.

For example, the automatic purification and dispensing apparatus 10 may include a purification unit that includes a multi-well plate kit (not shown) containing a reagent necessary for purification of the sample to purify the target nucleic acid from the sample and a pipette block 12 on which a plurality of pipettes 11 is detachably mounted to move the sample, the reagent, and the target nucleic acid.

In addition, the automatic purification and dispensing apparatus 10 may further include a pipette block driving unit (not shown) that drives the pipette block 12 so that the pipette block 12 is movable in at least one of an upward, downward, left, or right direction.

The purification unit may include a multi-well plate kit that has multiple columns and contains a sample for purifying the target nucleic acid and a reagent necessary for purifying the target nucleic acid in a specific column, a magnetic field applying part that applies a magnetic field to the specific column of the multi-well plate kit to separate magnetic particles attached to the target nucleic acid, and a heating part that applies heat to the specific column of the multi-well plate kit to accelerate the purification of the target nucleic acid.

The pipette block 12 is a configuration in which the plurality of pipettes 11 are detachably mounted to move the sample, the reagent, and the target nucleic acid used during the purification, and various configurations are possible.

For example, the pipette block 12 may have a plurality of insertion holes so that the plurality of pipettes 11 is inserted with the columns to suction and discharge the sample, the reagent, the target nucleic acid, and the like.

Here, the pipette block 12 may transfer the target nucleic acid obtained through the purification unit to the nucleic acid amplification test apparatus 20 through relative movement with the multi-well plate 40 to be described later.

In this process, the pipette block 12 may perform the relative movement with the multi-well plate 40, and a detailed relative movement process will be described later.

The pipette block driving unit may drive the pipette block 12 in at least one of the upward, downward, left, or right direction so that the plurality of pipettes 11 of the pipette block 12 move various samples, reagents, target nucleic acids, etc., to suction and discharge the various samples, reagents, target nucleic acids, and the like at a fixed position.

In the pipette block driving unit, various types of driving means disclosed in the related art may be applied, and for example, electric motor driving, hydraulic actuator driving, and the like may be used.

In the related art, after obtaining a target nucleic acid solution from the automatic purification and dispensing apparatus 10, the target nucleic acid solution may be put into the multi-well plate and then mixed using a manual pipette or an automatic pipette, and an inlet of the multi-well plate may be sealed manually and mounted manually on the nucleic acid amplification apparatus 20, and then, the nucleic acid amplification apparatus may be driven.

However, as described above, when the processes of mixing the target nucleic acid solution obtained from the automatic purification and dispensing apparatus 10 with the nucleic acid amplification kit and performing the sealing to move to the nucleic acid amplification test apparatus 20 are manually performed, there is a problem in that a risk of contamination and a total analysis time increase due to user's intervention.

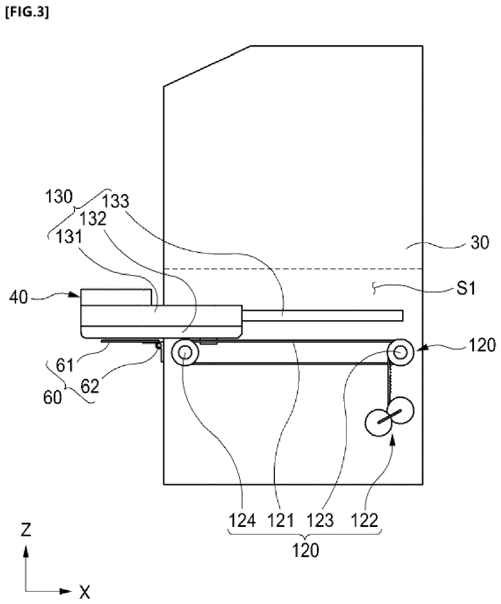

In order to solve this problem, as illustrated in FIGS. 3 to 5, the nucleic acid amplification test apparatus 20 according to the present invention may include a housing 30 having an inner space S1 and a multi-well plate insertion unit 100 which supports a multi-well plate 40 provided with a plurality of reaction tubes 1 and is installed to be movable between the dispensing position P and the inner space S1.

The housing 30 is configured to form the inner space S1, and various configurations are possible.

For example, the housing 30 may have a rectangular parallelepiped shape and form the inner space S1 isolated from the outer space, and the multi-well plate 40 provided with the plurality of reaction tubes 1 may be inserted and carried out.

For this, the multi-well plate insertion unit 100 may be provided with a shutter 60 on one surface thereof so that the multi-well plate insertion unit 100 supporting the multi-well plate 40 moves to be reciprocated between the inner space S1 and the dispensing position P for receiving the target nucleic acid through the automatic purification and dispensing apparatus 10.

More specifically, the housing 30 may be provided with a shutter 60 on one surface on which the multi-well plate insertion unit 100 is disposed so that the multi-well plate insertion unit 100 is movable between the inner S1 and the dispensing position P in the state in which the multi-well plate insertion unit 100 supports the multi-well plate 40.

Here, the shutter 60 may include a door part 61 forming a portion of one surface of the housing 30 and a door hinge 62 enabling opening and closing through rotation of the door part 61.

That is, when the door part 61 is pressed as the multi-well plate insertion unit 100 moves, the shutter 60 may be opened by the hinge rotation through the door hinge 62 of the door part 61, and the multi-well plate insertion unit 100 may move.

In the state in which the door part 61 is opened by the hinge rotation through the door hinge 62, the door part 61 may be maintained in the opened state through partial interference with the multi-well plate insertion unit 100.

As another example, in the state in which the door part 61 is opened by the hinge rotation through the door hinge 62, the rotation of the door part 61 may be stopped through a member fixing the door hinge 62, and then, the door part 61 may be maintained in the opened state.

In the state in which the multi-well plate 40 move to the dispensing position P of the automatic purification and dispensing apparatus 10 through the movement of the multi-well plate insertion unit 100, the target nucleic acid may be received through the automatic purification and dispensing apparatus 10 and then automatically move to the inner space S1 so that the amplification and detection of the nucleic acid are performed.

The multi-well plate insertion unit 100 may be a configuration that supports the multi-well plate 40 provided with the plurality of reaction tubes 1 and is movably installed between the dispensing position P and the inner space S1, and various configurations are possible.

For example, the multi-well plate insertion unit 100 may include a support part 110 configured to support an edge of the multi-well plate 40 in which the plurality of reaction tubes 1 are provided, and a driving part 120 configured to drive the support part 110 between the dispensing position P of the automatic purification and dispensing apparatus 10 and an amplification position, at which the amplification of the target nucleic acid is performed, in the housing 30.

In addition, the multi-well plate insertion unit 100 may further include a support driving block 130 provided at each of both lower ends of the support part 110 to connect the support part 110 to the driving part 120.

The multi-well plate 40 may be a configuration in which the plurality of reaction tubes 1 are formed in a plurality of columns, and various configurations are possible.

In particular, if the multi-well plate 40 is a configuration capable of accommodating the target nucleic acid to amplify and detect the target nucleic acid to be analyzed, any configurations disclosed in the related art may be applied.

For example, the multi-well plate 40 may be a configuration in which the plurality of reaction tubes 1 are coupled or supported to the plate 41, and as another example, the multi-well plate 40 may be a configuration in which a plurality of unit wells are formed on the plate 41 to accommodate the target nucleic acid through the wells.

The support part 110 may be a configuration that supports the edge of the multi-well plate 40 on which the plurality of reaction tubes 1 are provided, and various configurations are possible.

For example, the support part 110 may have an opening 111 in a center thereof to prevent interference with the reaction tube 1 of the multi-well plate 40 and include a stepped part 112 for supporting the multi-well plate 40.

That is, the support part 110 may be provided in the inner space S1 to support and move the multi-well plate 40 so that the multi-well plate 40 is disposed at a fixed position and more particularly moves to the dispensing position P at which the target nucleic acid is received from the automatic purification and dispensing apparatus 10.

The opening 111 may be a configuration provided so that the plurality of reaction tubes 1 pass through the support part 110 downward without interference and may be formed to correspond to a shape of the multi-well plate 40.

The plurality of reaction tubes 1 may be disposed at the lower side through the opening 111 in the state in which the plate 41 is supported on the stepped part 112 to control a temperature through the temperature control block 500.

The stepped part 112 may be a configuration for supporting the edge of the multi-well plate 40, and various configurations are possible.

The support driving block 130 may be a configuration provided at each of both the lower ends of the support 110 to connect the support part 110 to the driving part 120, and various configurations are possible.

The support driving block 130 may have an upper side coupled to the support part 110 at each of both the lower ends of the support part 110 and a lower side connected to the driving part 120 so that the support part 110 moves from the amplification position in the inner space S1 to the dispensing position P through driving force of the driving part 120.

More specifically, the support driving block 130 may include a support plate 131 coupled at each of both lower ends of the support part 110, a block part 132 connected to a driving belt 121 of the driving part 120 at a lower side of the support plate 131, and a driving guide 133 provided on the support plate 131.

Through this, since the support driving block 130 receives the driving force of the driving part 120 through the block part 132 to move, the driving force may be transmitted to the support part 110 connected to the support plate 131, as a result, the support driving block 130 may move between the amplification position and the dispensing position P together with the support part 110.

The drive guide 133 may be a configuration for guiding the movement of the support part 110 and may be provided on the support plate 131 to guide the movement of the support plate 131 and the support part 110 together with the guide member provided on an inner wall of the housing 30.

The driving part 120 may be a configuration that drives the support part 110 so as to be reciprocated between the inner space S1 of the housing 30 and the dispensing position P, and various configurations are possible.

For example, if the driving part 120 drives the support part 110 in the method disclosed in the related art, the driving part 120 may drive the support part through any method such as an electric motor type, a pneumatic type, a magnetic field linear motor, and the like.

For example, the driving part 120 may include a driving source 122 that receives external energy to perform rotational motion, a pair of pulley parts 123 and 124 connected to the driving source 122, and a driving belt 121 connected between the pair of pulley parts 123 and 124.

The driving source 122 may receive the external energy to perform the rotational motion as a motor and may rotate at least one of the pair of connected pulley parts 123 and 124 according to the rotational motion.

At least one of the pair of pulley parts 123 and 124 may rotate according to the rotation of the driving source 122, and the pair of pulley parts 123 and 124 may rotate vertically through the driving belt 121 to which the other one of the pair of pulley parts 123 and 124 is connected so that the driving belt 121 moves.

As described above, the driving belt 121 may be connected to the support plate 131 through the block part 132 of the support driving block 130, and as the driving belt 121 moves, the support plate 131 and the support part 110 may move.

The above-described movement may be performed in various directions like linear movement such as upward, downward, left, and right directions, and rotational movement, and more specifically, the side surface of the housing 30 of the nucleic acid amplification test apparatus 20 may linearly and horizontally move to allow the multi-well plate 40 to approach a side of the automatic purification and dispensing apparatus 10.

In the nucleic acid amplification test apparatus according to the related art, the user may directly seal a top surface of the multi-well plate 40 and transfer the multi-well plate 40 to the nucleic acid amplification test apparatus 20. Thus, there is a problem in that contamination occurs during the sealing process, constant sealing is difficult, and a total analysis time increases according to the sealing time to deteriorate analysis efficiency.

In order to solve these problems, the nucleic acid amplification test apparatus 20 according to the present invention may automatically receive the target nucleic acid obtained from the automatic purification and dispensing apparatus 10 and automatically perform the seal on the multi-well plate 40.

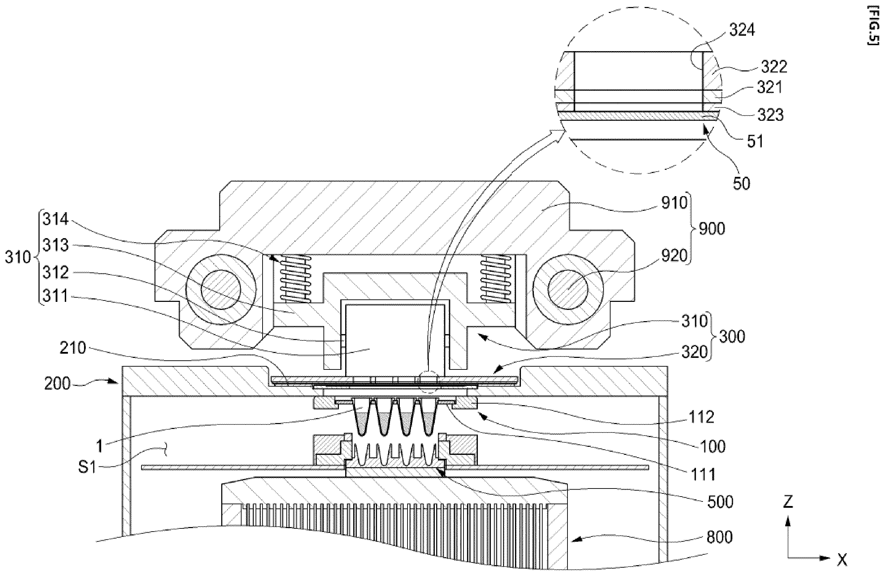

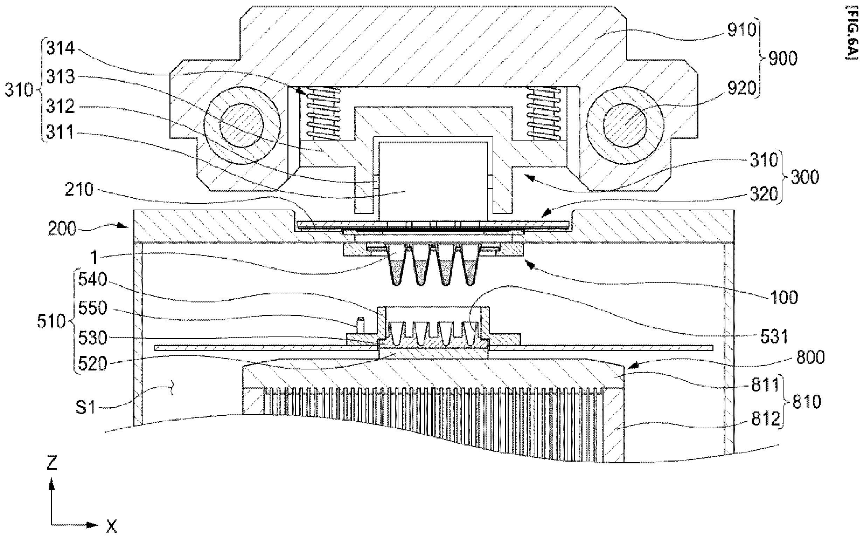

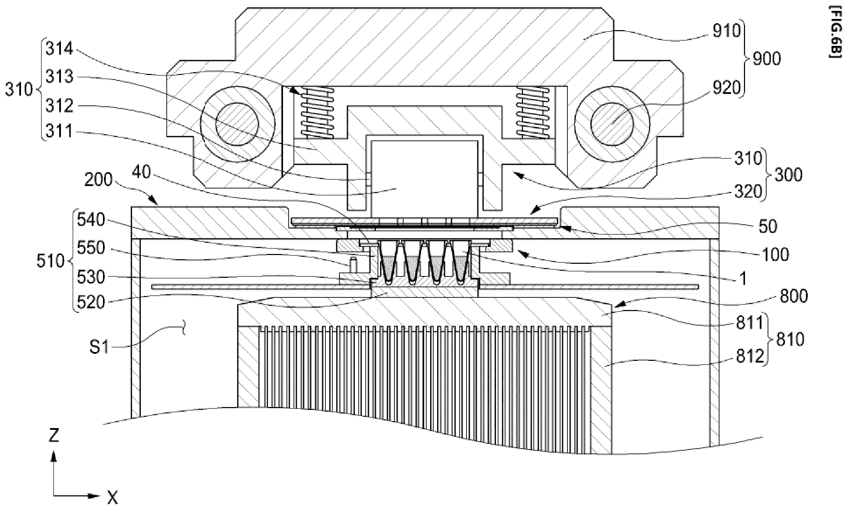

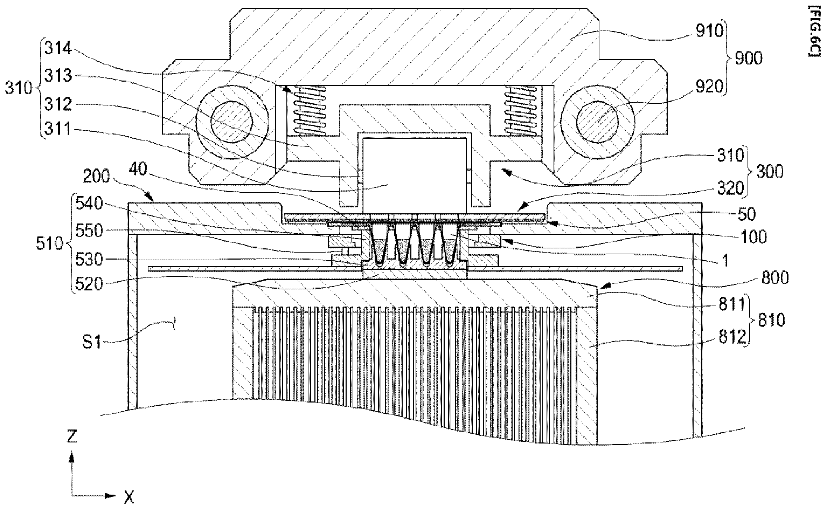

In more detail, as illustrated in FIGS. 5 and 6, the nucleic acid amplification test apparatus 20 includes: the housing 30 having the inner space S1 that is isolated from the outside; the multi-well plate insertion unit 100 configured to support the multi-well plate 40 provided with the plurality of reaction tubes 1, in which the target nucleic acid received through the automatic purification and dispensing apparatus 10 is accommodated; the sealing plate insertion unit 200 configured to support the sealing plate 50 provided with the sealing means for sealing the inlet of each of the plurality of reaction tubes 1 at the upper side of the multi-well plate 40; the automatic sealing unit 300 configured to relatively move the sealing means and the multi-well plate 40 so as to be adjacent to each other, thereby sealing the sealing means to the inlet of the reaction tube 1; the fluorescence detection unit 400 disposed above the sealing plate insertion unit 200 to detect the target nucleic acid in the reaction tubes 1; and the temperature control block 500 disposed below the multi-well plate insertion unit 100 to control a temperature of each of the reaction tubes 1.

Since the housing 30 and the multi-well plate support 100, which are portions of the configurations of the nucleic acid amplification test apparatus 20 according to the present invention, have been described above, detailed descriptions are omitted, and only portions requiring additional explanation are described with reference to the accompanying drawings.

Therefore, the contents of the above-described same configuration are applied to detailed configurations, which are not described for the housing 30 and the multi-well plate insertion unit 100 to be described below.

The housing 30 may be a configuration for forming the inner space S1 isolated from the outside and may include an insertion part 33 in which the multi-well plate 40 and the sealing plate 50 provided with the sealing means are inserted from the outside.

That is, the housing 30 may include an insertion part 33 that is opened to insert the multi-well plate 40 and the sealing plate 50 provided with the sealing means into the inner space S1 isolated from the outside.

The insertion part 33 may be opened and formed in a size corresponding to the sealing plate 50 and the multi-well plate 40 at one side of the top surface of the housing 30, and the above-mentioned support part 110 may be disposed to be slid and inserted while supporting an edge of the multi-well plate 40.

As described above, in the insertion part 33, a sealing plate insertion unit 200 to be described later may be disposed to be slid and inserted while supporting the edge of the sealing plate 50.

The multi-well plate insertion unit 100 may be a configuration that supports the multi-well plate 40 provided with the plurality of reaction tubes 1, in which the target nucleic acid received through the automatic purification and dispensing apparatus 10 is accommodated, and various configurations are possible.

The sealing plate insertion unit 200 may be a configuration that supports the sealing plate 50 provided with the sealing means for sealing the inlets of the plurality of reaction tubes 1 at the upper side of the multi-well plate 40, and various configurations are possible.

In addition, the sealing means according to the present invention may be a sealing film 51 or a sealing cap 52 that seals the inlet of the reaction tube 1.

The sealing film 51 may be a configuration that seals the inlet of the reaction tube 1 to prevent the target nucleic acid from being evaporated during the process of repeatedly heating and cooling the target nucleic acid accommodated in the reaction tube 1 at an appropriate temperature through the temperature control block 500, and various configurations are possible.

The sealing film 51 may be formed and disposed in a size corresponding to the multi-well plate 40 in which the plurality of reaction tubes 1 are disposed in a plurality of columns.

In addition, the sealing cap 52 may be a configuration that seals the inlet of the reaction tube 1 to prevent the target nucleic acid from being evaporated during the process of repeatedly heating and cooling the target nucleic acid accommodated in the reaction tube 1 at an appropriate temperature through the temperature control block 500, and various configurations are possible.

For example, the sealing cap 52 may be provided in plurality to have a size corresponding to a position corresponding to the inlet of each of the reaction tubes 1 of the multi-well plate 40 in which the plurality of reaction tubes 1 are disposed in the plurality of columns.

Although the sealing film 51 is described below as a main embodiment, it is also possible to apply the configuration of the sealing film 51 to the sealing cap 52.

The sealing plate 50 may be a plate provided with the sealing film 51 and may be disposed at a fixed position above the multi-well plate 40 through the insertion part 33 of the housing 30 described above.

The sealing plate insertion unit 200 may be provided above the multi-well plate insertion unit 100 in the inner space S1 to support the sealing plate 50, and various configurations are possible.

A center of the sealing plate insertion unit 200 may be opened so that the plurality of reaction tubes 1 provided in the multi-well plate 40 are in contact with the lower side of the sealing film 51, and the sealing plate insertion unit 200 may include a support stepped part 210 for supporting the edge of the sealing plate 50.

Particularly, the sealing plate insertion unit 200 may be formed in a shape corresponding to the shape of the sealing plate 50, and an end thereof may be formed adjacent to the insertion part 33 so that the sealing plate 50 is slid, inserted, and supported through the support stepped part 210.

Identification information such as a barcode or a QR code that are capable of being sensed through a measurement sensor 550 to be described later may be provided on the sealing plate insertion unit 200 or the sealing plate 50.

As a result, it is possible to identify whether the sealing film 51 is provided through the measurement sensor 550 to be described later to prevent an operation from proceeding even when the sealing film is missing, and thus, a test and analysis defect rate may be reduced.

The automatic sealing unit 300 may be a configuration that relatively moves the sealing film 51 and the multi-well plate 40 to be adjacent to each other so that the sealing film 510 is sealed to the inlet of the reaction tube 1, and various configurations are possible.

For example, the automatic sealing unit 300 may include a pressing part 310 that is provided to be movable above the sealing plate inserting unit 200 so as to directly or indirectly press the sealing film 51 toward the multi-well plate 40.

In addition, the automatic sealing unit 300 may further include a heating part 320 provided between the pressing part 310 and the sealing plate seated on the sealing plate insertion unit 200 to create high-temperature environments at an inlet-side of the reaction tube 1 of the multi-well plate 40.

The pressing part 310 may be provided to be movable above the sealing plate insertion unit 200 so as to directly or indirectly press the sealing film 51 toward the multi-well plate 40, and various configurations are possible.

That is, the pressing part 310 may press the sealing plate 50 toward the multi-well plate 40 in a state in which the sealing plate 50 and the multi-well plate 40 are spaced apart from each other so that the sealing film 51 seals the inlets of the reaction tubes 1.

For example, the pressing part 310 may include a pressing roller 311 configured to seal the sealing means to the inlet of the reaction tube 1 through horizontal movement rolling in the state of directly or indirectly pressing the sealing means at the upper side of the sealing plate insertion unit 200, and a roller driving part 900 configured to drive the pressing roller 311.

In addition, the pressing part 310 may further include a pressing roller shaft 312 installed across both ends from a center of the pressing roller 311 and a pressing roller body 313 in which the pressing roller shaft 312 is installed.

In addition, the pressing part 310 may include an elastic connection part 314 to be elastically connected to a driving body 910 of the roller driving part 900 to be described later.

That is, in the pressing roller body 310, the pressing roller body 313 may be connected to the roller driving part 900 through an elastic connection part 314, and the pressing roller shaft 312 and the pressing roller 313 may be provided to rotate and move the pressing roller 313.

In this process, the roller driving part 900 and the pressing roller body 313 may be connected to each other through the elastic connection part 314 to prevent excessive pressing from being applied to the sealing plate 40 while pressing the sealing plate 40 through the pressing roller 311.

The roller driving part 900 may include a driving body 910 connected to the pressing roller body 313 through the elastic connection part 314, a driving shaft 920 forming a moving path of the pressing roller 311 to guide movement of the driving body 910, a pressing driving belt part 940 moving the driving body 910 through rotational movement of the pressing driving belt 941 because the pressing driving belt 941 is connected to the driving body 910, and a pressing driving source 930 constituting the pressing driving belt part 940 to move the driving body 910.

More specifically, the pressing driving belt part 940 may include pressing driving belts 941 connected to the driving body 910 in parallel to the driving shaft 920 with respect to the driving shaft 920 connected to the driving body 910 to guide the movement of the driving body 910 and a pair of pressing driving pulleys 942 provided on both ends on which the pressing driving belts 941 are rotatably provided.

As a result, the rotational motion of the pressing roller driving source 943, which is a motor, may be transmitted to each of the pressing drive pulleys 942, and one pressing drive pulley 942 may rotate to rotate the pressing driving belts 941 with respect to the pair of pressing drive pulleys 942, and thus, the driving body 910 connected to the pressing driving belt 941 may be guided to the driving shaft 920 to linearly move.

In order to drive the pressing driving belt 940 provided on each of both sides with respect to the driving shaft 920 through one pressing driving source 930, the roller driving part 900 may further include a driving transmission shaft 950 provided across the pair of driving shafts 920 to transmit the rotational force transmitted from the pressing driving source 930 to each of the pressing driving pulleys 942.

The heating part 320 may be a configuration that is provided between the pressing part 310 and the sealing plate 50 seated on the sealing plate insertion unit 200 to create high-temperature environments at an inlet-side of the reaction tube 1 of the multi-well plate 40, and various configurations are possible.

For example, the heating part 320 may be a heater provided between the pressing part 310 and the sealing plate 50 seated on the sealing plate insertion unit 200 and may create the high-temperature environments at the inlet-side of the reaction tube 1 to prevent dew condensation from occurring on the inlet-side of the reaction tube 1.

In addition, the heating part 320 may serve to reinforce the sealing of the sealing means with respect to the inlet of the reaction tube 1 by heating the sealing means according to a material of the sealing means.

As a result, the heating part 320 may prevent the dew condensation or moisture from occurring on the inlet-side of the reaction tube 1 to irradiate light through the fluorescence detection unit 400 to be described later and prevent the light from being blocked, reflected, and scattered so that fluorescence detection is performed smoothly.

More specifically, the heating part 320 may include a heater 321 for transferring heat to the inlet of the reaction tube 1, a heater upper cover 322 protecting the heater 321 from the upper side, and a heater lower cover 323 supported and disposed on the sealing plate insertion unit 200 below the heater 321.

The heater 321 may be a configuration for transferring the heat to the upper portion of the reaction tube 1 and may prevent condensation and moisture from occurring on the inlet-side of the reaction tube 1.

The heater upper cover 322 may be a configuration that is provided above the heater 321 to protect the heater 321 and the sealing film 51 in the process of pressing the sealing film 51 through the pressing part 310, and various configurations are possible.

In particular, the heater upper cover 322 may be disposed above the heater 321 to transmit the pressing force of the pressing part 310 to the sealing film 51 through the heater 321 so that the sealing film 51 is in close contact with the upper portion of the multi-well plate 40 including the plurality of reaction tubes 1.

That is, the heater upper cover 322 may prevent direct pressing of the sealing film 51 through the pressing part 310 and indirectly press the sealing film 51 to prevent the sealing film 51 from being damaged and also apply appropriate pressing force to the sealing film 51 so that the sealing film 51 is in close contact with and in contact with the upper side of the multi-well plate 40.

The heating part 320 may have through-holes 324 corresponding to the plurality of reaction tubes 1 to detect the target nucleic acid through the fluorescence detection unit 400 disposed above the sealing plate insertion unit 200 with respect to the reaction tubes 1.

Here, the through-holes 324 may be formed in the heating part 320 including the heater upper cover 322 and the heater 321 to detect the target nucleic acid accommodated in the reaction tube 1 through the light irradiation of the fluorescence detection unit 400 to be described later.

In particular, the through-holes 324 are formed to be aligned at a position at which the plurality of reaction tubes 1 are disposed among the upper heater cover 322 and the heater 321, and thus, the target nucleic acid may be detected through the light passing through the upper heater cover 322 and the heater 321.

For this purpose, the heating part 320 including the heater upper cover 322 and the heater 321 may have elasticity so that the pressing force through the pressing part 310 is transmitted to the sealing film 51.

The fluorescence detection unit 400 may be a configuration that is disposed above the sealing plate insertion unit 200 to detect the target nucleic acid in the reaction tube 1, and various configurations are possible.

For example, the fluorescence detection unit 400 may include a detection part 430 configured to irradiate excitation light with which the target nucleic acid reacts toward the reaction tube 1 and sense fluorescence according to the reaction of the target nucleic acid, and a detection driving part 440 configured to drive the detection part 430 to be movable above the sealing plate insertion unit 200.

That is, the fluorescence detection unit 400 may measure the target nucleic acid contained in the reaction tube 1 in real-time by irradiating excitation light to which the target nucleic acid accommodated in the reaction tube 1 reacts and sensing the resulting fluorescence.

The detection part 430 may be a configuration that irradiates light reacting with the target nucleic acid toward the reaction tube 1 to sense fluorescence of the target nucleic acid, and various configurations are possible.

For example, the detection part 430 may include a light source part 410 configured to irradiate the excitation light, which the target nucleic acid reacts, toward the reaction tube 1, and a detection sensor 420 configured to sense the fluorescence generated in proportion to a concentration of the target nucleic acid through the irradiated light.

In this case, the detection part 430 may be a configuration that is provided with a light source part 410 configured to irradiate specific excitation light to each target nucleic acid toward the reaction tube 1 and a detection sensor 420 configured to sense generated fluorescence as an integrated module, and for another example, the light source part 410 and the detection sensor 420 may be provided separately.

The light source part 410 may be a configuration that irradiates light having a specific wavelength at which the target nucleic acid reacts toward the reaction tube 1, and the light having a target wavelength range may be variously applied according to the type of fluorescent probe specific to the target nucleic acid to be analyzed.

Here, the light source part 410 may be disposed above the heating part 320 to irradiates the excitation light so as to reach the reaction tube 1 through the through-hole 324 provided in the heating part 320.

For this, the light source part 410 may be fixedly disposed at a position that is vertically aligned with the reaction tube 1 and the through-hole 324 on the upper end of the housing 30. For another example, the light source part 410 may be driven through a detection driving part 440 to be described later and then be disposed in a fixed position.

The detection sensor 420 may be a configuration that senses the fluorescence generated through excitation light irradiated from the light source part 410 to the target nucleic acid in the reaction tube 1, and various configurations are possible.

The detection sensor 420 may irradiate excitation light having a specific wavelength range to detect the fluorescence generated in proportion to the concentration of the target nucleic acids, thereby measuring an amount of target nucleic acids in real-time, and like the light source part 410, the detection sensor 420 may be fixedly arranged at a position vertically aligned with the through-hole 324 or may move together with the light source part 410 through the detection driving part 440 so as to be disposed at the fixed position.

The detection driving part 440 may be a configuration that drives the detection part 430 to be movable from the upper side of the sealing plate inserting unit 200, and various configurations are possible.

For example, the detection driving part 440 may be provided to drive independently of the pressing part 310 through a separate driving source while sharing the above-described roller driving part 900 and the driving shaft 920, which are described above.

That is, the detection driving part 440 may be connected to the drive shaft 920 of the roller driving part 900 to move in a longitudinal direction of the nucleic acid amplification test apparatus 20 and the sealing plate 50.

Furthermore, the detection driving part 440 may further include components for driving the nucleic acid amplification test apparatus 20 and the sealing plate 50 in a vertical direction and a vertical direction with respect to the longitudinal direction.

For example, the detection driving part 440 may include a detection driving block 444 provided with a detection part 430, a pair of detection driving guides 442 and 443 that guide movement of the detection driving block 444, a pair of detection driving belts 445 and 446 that are connected to the detection driving block 444 to move the detection driving block 444, a pair of detection driving belts that move the pair of detection driving belts 445 and 446, and a detection driving source 441 that drives each of the pair of detection driving belts 445 and 446.

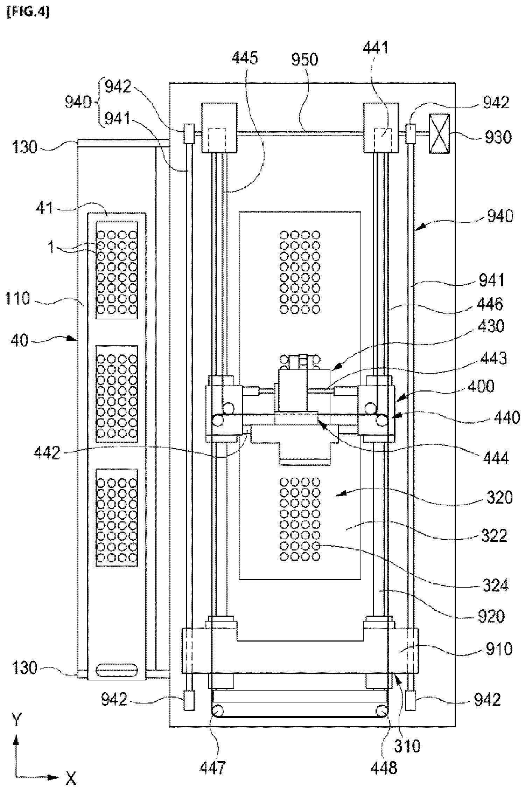

The detection driving part 440 will be described in detail with reference to FIG. 4.

Two detection driving sources 441 are provided at ends of the driving shaft 910 to drive the detection driving belts 445 so as to rotate, respectively.

In addition, the pair of detection drive pulleys 447 and 448 may be provided at opposite ends of the detection driving source 441 to guide rotation of the pair of detection driving belts 445 and 446.

In this case, the pair of detection drive pulleys 447 and 448 may be disposed at ends of the pair of driving shafts 910, and thus, the pair of detection driving belts 445 and 446 may be disposed on edges of the driving shaft 910, respectively.

One detection driving belt 445 may be connected to one corresponding detection driving source 441 to rotate, a path of the detection driving belt 445 may be connected to one side of the driving shaft 910 in parallel to the driving shaft 910 between the detection part 430 and the detection driving source 441 with respect to the detection part 430, and the pair of detection driving pulleys 447 and 448 and the pair of driving shafts 910 may be disposed at the outside at the opposite ends of the detection part 430 and the driving shaft 910 and be disposed in a rectangular shape on a plane.

That is, one detection driving belt 445 may be connected to one detection driving source 441 to pass through the detection drive pulley 447 disposed at the end along the drive shaft 910, thereby reaching the other detection driving pulley 448 disposed parallel thereto and then may be converted in direction using the detection driving pulley 448 so as to be connected to one detection driving source 441 in a state of being disposed across the driving shaft 910 so as to be connected to the detection driving block 444.

That is, the other detection driving belt 446 may be connected to the other detection driving source 441 facing the above-described detection driving source 441 to pass through the detection drive pulley 448 disposed at the end of the driving shaft 910 along the opposite drive shaft 910, thereby reaching the other detection driving pulley 447 and then may be converted in direction using the detection driving pulley 447 so as to be connected to the other detection driving source 441 in a state of being disposed across the driving shaft 910 so as to be connected to the detection driving block 444.

As a result, the detection driving part 440 may freely move the detection part 430 in an X direction and a Y direction on the plane of FIG. 4 and may be driven independently of the above-described pressing part 310.

The detection driving part 440 may move the detection part 430 in the vertical direction in the same manner as described above.

The detection driving part 440 may constitute an independent driving part separately from the above-described embodiment in which the detection driving part 440 linearly moves in the longitudinal direction through the roller driving part 900, and thus, the detection part 430 may linearly move in the longitudinal direction at the upper side of the housing 30.

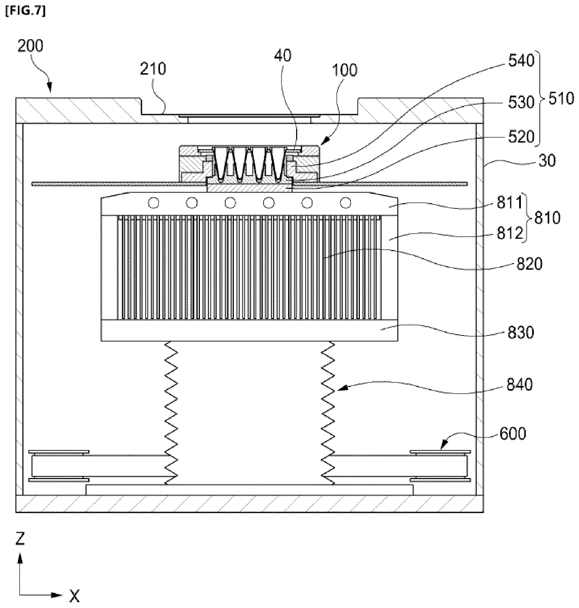

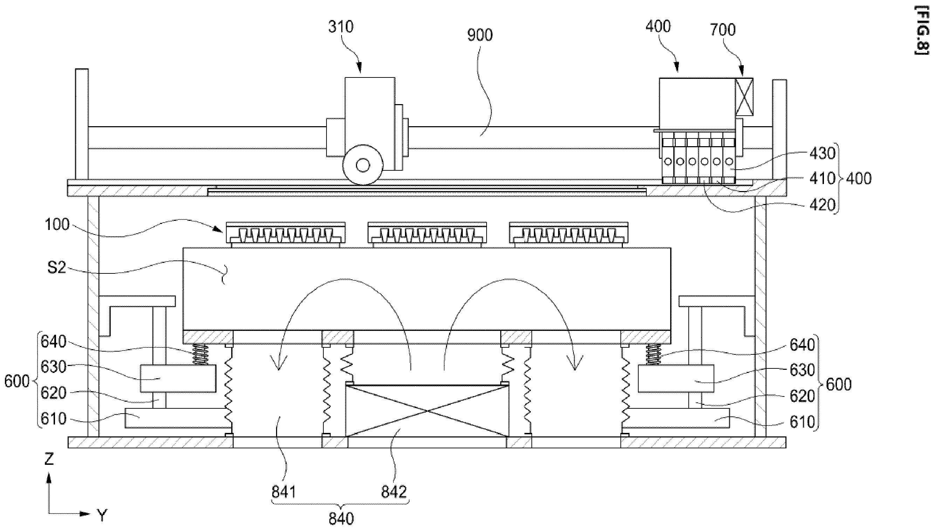

As illustrated in FIGS. 7 and 8, the temperature control block 500 may be a configuration that is disposed below the multi-well plate insertion unit 100 to control a temperature of the target nucleic acid in the reaction tube 1, and various configurations are possible.

The temperature control block 500 may include a heating block 510 disposed below the multi-well plate insertion unit 100 to control the temperature of the reaction tube 1 by performing heat-exchange with the reaction tube 1, and a heat dissipation unit 800 provided below the heating block 510 to transfer heat generated from the heating block 510 to the outside.

In addition, the temperature control block 500 may further include an elevation driving unit 600 that drives the heat dissipation unit 800 vertically to move the heating block 510 supporting the multi-well plate 40 vertically, thereby vertically driving the position of the multi-well plate 40.

The heating block 510 may be a configuration that is disposed below the multi-well plate insertion unit 100 to control the temperature of the reaction tube 1 by exchanging heat with the reaction tube 1, and various configurations are possible.

For example, the heating block 510 may include a thermoelectric module 520 and a reaction block 530 having a bottom surface that is in a surface contact with the thermoelectric module 520 and a top surface, in which a plurality of insertion grooves 531 into which the plurality of reaction tubes 1 are inserted are formed.

In addition, the heating block 510 may further include a reaction cover 540 coupled to the reaction block 530 so as to surround an outer surface of the reaction block 530.

In addition, the heating block 510 may include a measurement sensor 550 configured to sense the sealing plate 50 disposed thereabove so as to identify whether the sealing plate 50 exists.

The heating block 510 may be a configuration that is disposed below the multi-well plate insertion unit 100 to control the temperature of the reaction tube 1 by exchanging heat with the reaction tube 1, thereby amplifying the nucleic acid in the reaction tube 1, and various configurations are possible.

In particular, the heating block 510 may induce the amplification of the target nucleic acid by repeatedly performing a combination of heating and cooling as a unit cycle. Here, the specific details of the temperature control may be variously applied according to the type of the target nucleic acid.

The thermoelectric module 520 may be a configuration that controls the temperature, the heating, and the cooling according to application of current and its direction, and various configurations are possible.

In particular, the thermoelectric module 520 may be applied to any type of thermoelectric module 520 disclosed in the related art and may be disposed to correspond to a bottom surface of the reaction block 530 that supports the reaction tubes 1 below the multi-well plate 40 provided with the reaction tubes 1.

The heating block 530 may be a configuration having a bottom surface that is in a surface contact with the thermoelectric module 520 and a top surface, in which a plurality of insertion grooves 531 into which the plurality of reaction tubes 1 are inserted are formed, and various configurations are possible.

Here, the reaction block 530 may be disposed above the thermoelectric module 520 and be provided with insertion grooves 531 so that at least a portion of the reaction tube 1, more preferably, the accommodated target nucleic acid is completely inserted at positions disposed corresponding to the reaction tubes 1.

As a result, the reaction block 530 may allow a portion of a lower side of the reaction tube 1 to be inserted into the insertion groove 531 so that the heat through the thermoelectric module 520 is heat-exchanged with the reaction tube 1, particularly a position of the reaction tube 1, in which the target nucleic acid is disposed.

Furthermore, the reaction block 530 may support the reaction tubes 1 to allow the reaction tubes 1 to ascend when ascending through an elevation driving unit 600 to be described later, and thus, the inlet of the reaction tube 1 reaches a position adjacent to the sealing film.

The reaction cover 540 may be a configuration to be coupled to the reaction block 530 so as to surround the outer surface of the reaction block 530, and various configurations are possible.

Here, the reaction cover 540 may be coupled to an edge of the reaction block 530 so that the heat transferred through the thermoelectric module 520 is transferred to the reaction block 530 without being dissipated to the side of the reaction block 530, thereby improving thermal efficiency.

In addition, the reaction cover 540 may support the plate 41 of the multi-well plate 40 to ascend when ascending through the elevation driving unit 600 to be described later.

The measurement sensor 550 may be a configuration that senses the sealing plate 50 disposed thereabove so as to identify whether the sealing plate 50 exists, and various configurations are possible.

For example, the measurement sensor 550 may be provided outside the reaction cover 540 to irradiate light toward the upper sealing plate 50 or the sealing plate insertion unit 200, thereby sensing the reflected light so as to identify whether the sealing film 51 exists.

As another example, when the measurement sensor 550 is provided outside the reaction cover 540 and is provided with identification information such as a barcode or QR code that is capable of being sensed on the upper sealing plate 50 or the sealing plate insertion unit 200, a degree of identification may be recognized to identify the presence or absence and related information.

The heat dissipation unit 800 may be a configuration provided below the heating block 510 to transfer the heat generated in the heating block 510 to the outside, and various configurations are possible.

For example, the heat dissipation unit 800 may include a heatsink 810 including a heat dissipation plate 811 provided to be in surface contact with a lower side of the heating block 510 and a plurality of heat dissipation fins 812 provided on the heat dissipation plate 811; and a plurality of side surface parts 820 and bottom surface parts 830 coupled to the heat dissipation plate 811 so that the plurality of heat dissipation fins 812 are disposed in a separation space S2 that is separated from the inner surface S1 to form the separation space S2 together with the heat dissipation plate 811.

In addition, the heat dissipation unit 800 may further include an air circulation part 840 provided to independently communicate with the separation space S2 and the outside of the housing 30 so that internal air of the separation space S2 is circulated with external air of the housing 30.

The heatsink 810 may be a configuration provided to be in contact with the thermoelectric module 520 below the heating block 510 to transfer the heat generated in the heating block 510 to the outside, and various configurations are possible.

For example, the heatsink 810 may include a heat dissipation plate 811 provided to be in surface contact with the lower side of the heating block 510 and a plurality of heat dissipation fins 812 provided on the heat dissipation plate 811.

The heatsink 810 may be a configuration that is disposed below the thermoelectric module 520 so that the plurality of heat dissipation fins 812 are disposed therebelow, and any type of heatsink 810 disclosed in the related art may be applied.

In particular, the heat dissipation plate 811 may have a refrigerant path 813 formed therein to continuously exchange heat through the refrigerant, thereby maximizing heat dissipation efficiency.

Here, the refrigerant path 813 and a refrigerant may be applied to various types of refrigerants disclosed in the related art.

In addition, the heat dissipation fins 812 may be provided in plurality in the heat dissipation plate 811 to improve heat dissipation efficiency, and various configurations are possible.

In particular, the heat dissipation fin 812 may be provided in a simple fin shape, but more preferably, may be a parallel planar fin having an area.

The heat dissipation fin 812 may be disposed parallel to a flow direction of air for smooth air circulation in the air circulation part 840 to be described later.

The side surface part 820 and the bottom surface part 830 may be configurations that are coupled to the heat dissipation plate 811 so that the plurality of heat dissipation fins 812 are disposed in the separation space S2 separated from the inner space S1 to form the separation space S2 together with the heat dissipation plate 811, and various configurations are possible.

That is, in the side surface part 820 and the bottom surface part 830, the side surface part 820 may be coupled to a lower edge of the heat dissipation plate 811, and the bottom surface part 830 may be coupled to a lower side of each of the plurality of side surface parts 820 so that the plurality of heat dissipation fins 812 therein are disposed in the separate separation space S2 that is separated from the inner space S1.

As a result, it is possible to form the separation space (S2) in which the heat dissipation fin 812 is disposed so that the air circulation through the air circulation part 840 is possible.

The air circulation part 840 may be a configuration provided to independently communicate with the separation space S2 and the outside of the housing 30 so that internal air of the separation space S2 is circulated with external air of the housing 30, and various configuration is possible.

For example, the air circulation part 840 may include a plurality of connection tubes 841 connected between the bottom surface part 830 and the housing 30, and an air circulation fan 842 installed in at least one of the plurality of connection tubes 841 to suction air outside the housing 30 or discharge air inside the separation space S2.

That is, the air circulation part 840 may be a configuration that directly communicates with the outside of the housing 30 and the separation space S2 formed through the above-described side surface parts 820 and the bottom surface part 830 to induce continuous air circulation in the separation space S2, and various configurations are possible.

For example, the air circulation part 840 may be connected to the plurality of connection tubes 841 connected between the bottom surface part 830 and the housing 30 and thus may be separated from the inner space S1 so that the separation space S2 independently communicates with the outside of the housing 30.

In this case, each of the connection tubes 841 may be provided as a corrugated tube or bellows to maintain the separation state between the separation space S2 and the inner space S1 when the entire heat dissipation unit 800 ascends and descends through the elevation driving unit 600 to be described later.

The connection tubes 841 are more preferably provided in three and may be connected between the bottom surface portion 830 and the housing 30.

The air circulation fan 842 is installed in at least one of the plurality of connection tubes 841 to suction air outside the housing 30 or discharge air inside the separation space S2.

More specifically, the air circulation fan 842 may be installed in one of the plurality of connection tubes 841 to suction the air outside the housing 30, thereby discharging the air within the separation space S1 through the other connection tube 841, in which the air circulation fan 842 is not installed.

In addition, the air circulation fan 842 may be installed in one of the plurality of connection tubes 841 to discharge the air outside the housing 30, thereby injecting the air outside the housing 30 into the separation space S2 through the other connection tube 841 in which the air circulation fan 842 is not installed.

As illustrated in FIGS. 6A to 6C, the elevation driving unit 600 may be a configuration that drives the heat dissipation unit 800 vertically to move the heating block 510 supporting the multi-well plate 40 vertically so that the position of the multi-well plate 40 moves vertically, and various configurations are possible.

In this case, the elevation driving unit 600 may simply elevate the heat dissipation unit 800 to support the reaction tubes 1 of the multi-well plate 40 supported by the multi-well plate insertion unit 100 through the heating block 510.

Furthermore, the elevation driving unit 600 may elevate the multi-well plate 40 supported by the multi-well plate insertion unit 100 through the heating block 510 by the elevation of the heat dissipating unit 800 to ascend to a position adjacent to the sealing film 51 so as to be supported.

In this case, the elevation driving unit 600 may support the lower side of the reaction tube 1 during the pressing process of the above-described pressing part 310, thereby improving the degree of completion of sealing.

For another example, the elevation driving unit 600 may elevate the heat dissipation unit 800 to allow the multi-well plate 40 to move so that the plurality of reaction tubes 1 are attached to the sealing film 51.

That is, as described above, the elevation driving unit 600 may allow the multi-well plate 40 to ascend up to the sealing film plate 50 through the ascending of the heat dissipation unit 800, so that the inlet of the reaction tube 1 is in contact with the sealing film 51 to seal the sealing film 51 on the reaction tube 1.

The elevation driving unit 600 may be a configuration that elevates the heat dissipation unit 800, and various elevation driving methods disclosed in the related art may be applied.

For example, the elevation driving unit 600 may perform the elevation through various methods such as a pulley method using an electric motor, a ball screw linear movement method, a hydraulic drive method, and a linear drive method using an electromagnetic field.

More specifically, the elevation driving unit 600 may include a rotation driving source 610 rotating through external energy, an elevation rotation shaft 620 rotating by being connected through the rotation driving source 610, an elevation driving connection block 630 connected to the elevation rotation shaft 620, and an elevation guide 640 connected to the elevation driving connection block 630 and the heat dissipation unit 800, more particularly, to the bottom surface part 830.

As a result, the elevation driving unit 600 may rotate the elevation rotation shaft 620 through the rotation driving source 610 to vertically move the elevation driving connection block 630 screw-coupled to the elevation rotation shaft 620, and thus, the bottom surface part 830 may move along the elevation guide 640.

The scan unit 700 may scan an identification information code attached to the reaction tube 1 to acquire target nucleic acid information, and various configurations are possible.

For example, the scan unit 700 may be provided together with the above-described fluorescence detection unit 400 or separately provided at the upper side of the housing 30 to scan the identification information code attached to the reaction tube 1 and acquire and identify information of the target nucleic acid.

Furthermore, the scan unit 700 may scan a position information code attached to the sealing plate 50 to further identify whether the sealing film 51 exists.

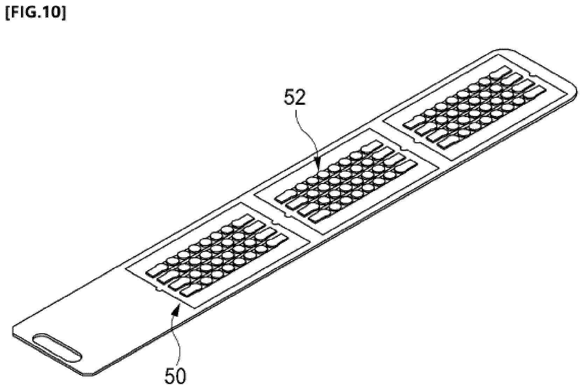

As illustrated in FIG. 10, the sealing means according to the present invention, in particular, the sealing cap 52 may be provided to correspond to, for example, the number of reaction tubes 1, and more specifically, eight sealing caps may be provided to correspond to eight reaction tubs 1, and a handle for carrying the sealing cap 52 by the user may be provided at each of both ends of the sealing cap 52.

That is, the eight sealing caps 52 may be provided in a row, and the handle may be provided on the sealing cap 52 disposed on each of both the ends to extend.

Although the above description merely corresponds to some exemplary embodiments that may be implemented by the present invention, as well known, the scope of the present invention should not be interpreted as being limited to the above-described embodiments, and all technical spirits having the same basis as that of the above-described technical spirit of the present invention are included in the scope of the present invention.

## Claims

1. A nucleic acid amplification test apparatus of an automatic sample analysis system which comprises an automatic purification and dispensing apparatus (10) configured to purify and dispense a target nucleic acid that is an analysis target, and a nucleic acid amplification test apparatus (20) configured to amplify and measure acquired from the automatic purification and dispensing apparatus (10), the nucleic acid amplification test apparatus comprising:
a housing (30) having an inner space (S1) that is isolated from the outside;
a multi-well plate insertion unit (100) in which a multi-well plate (40) provided with a plurality of reaction tubes (1), in which a target nucleic acid solution is accommodated, is inserted through an automatic purification and dispensing apparatus (10);
a sealing plate insertion unit (200) into which a sealing plate (50) provided with a sealing means for sealing inlets of the plurality of reaction tubes (1);
a fluorescence detection unit (400) disposed above the sealing plate insertion unit (200) to detect the target nucleic acid in the reaction tubes (1); and
a temperature control block (500) disposed below the multi-well plate insertion unit (100) to control a temperature of each of the reaction tubes (1),
wherein the reaction tubes (1) relatively move to be adjacent to the sealing means so as to be sealed by the sealing means.

2. The nucleic acid amplification test apparatus of claim 1, wherein the temperature control block (500) allows the reaction tubes (1) to ascend to be in closely contact with the sealing unit through elevation driving so that the reaction tubes (1) are sealed by the sealing means.

3. The nucleic acid amplification test apparatus of claim 1, wherein the temperature control block (500) comprises:
a heating block (510) comprising a thermoelectric module (520) and disposed below the multi-well plate insertion unit (100) to control the temperature of the reaction tube (1) by exchanging heat with the reaction tube (1) ;
a heat dissipation unit (800) provided below the heating block (510) to discharge the heat generated in the thermoelectric module (520) to the outside;
an elevation driving unit (600) configured to drive the heat dissipation unit (800) vertically to allow the heating block (510) configured support the multi-well plate (40) to move vertically so as to drive a position of the multi-well plate (40) vertically.

4. The nucleic acid amplification test apparatus of claim 3, wherein the heating block (510) comprises the thermoelectric module (520) and a reaction block (530) having a bottom surface that is in a surface contact with the thermoelectric module (520) and a top surface, in which a plurality of insertion grooves (531) into which the plurality of reaction tubes (1) are inserted are formed.

5. The nucleic acid amplification test apparatus of claim 3, wherein the heating block (510) further comprises a reaction cover (540) coupled to the reaction block (530) so as to surround an outer surface of the reaction block (530).

6. The nucleic acid amplification test apparatus of claim 3, wherein the heating block (510) comprises a measurement sensor (550) configured to sense the sealing plate (50) disposed thereabove so as to identify whether the sealing plate (50) exists.

7. The nucleic acid amplification test apparatus of claim 3, wherein the heat dissipation unit (800) comprises:
a heatsink (810) comprising a heat dissipation plate (811) provided to be in surface contact with a lower side of the thermoelectric module (520) and a plurality of heat dissipation fins (812) provided on the heat dissipation plate (811); and
a plurality of side surface parts (820) and bottom surface parts (830) coupled to the heat dissipation plate (811) so that the plurality of heat dissipation fins (812) are disposed in a separation space (S2) that is separated from the inner surface (S1) to form the separation space (S2) together with the heat dissipation plate (811).

8. The nucleic acid amplification test apparatus of claim 7, wherein the heat dissipation unit (800) comprises an air circulation part (840) provided to independently communicate with the separation space (S2) and the outside of the housing (30) so that internal air of the separation space (S2) is circulated with external air of the housing (30).

9. The nucleic acid amplification test apparatus of claim 8, wherein the air circulation part (840) comprises:
a plurality of connection tubes (841) connected between the bottom surface part (830) and the housing (30); and
an air circulation fan (842) installed in at least one of the plurality of connection tubes (841) to suction air outside the housing (30) or discharge air inside the separation space (S2).

10. The nucleic acid amplification test apparatus of claim 3, wherein the elevation driving unit (600) drives the heat dissipation unit (800) to allow the multi-well plate (40) to move so that the plurality of reaction tubes (1) are attached to the sealing means.

11. The nucleic acid amplification test apparatus of claim 1, wherein the multi-well plate insertion unit (100) comprises:
a support part (110) configured to support an edge of the multi-well plate (40) in which the plurality of reaction tubes (1) are provided; and
a driving part (120) configured to drive the support part (110) between a dispensing position (P) of the automatic purification and dispensing apparatus (10) and an amplification position in the housing (30).

12. The nucleic acid amplification test apparatus of claim 1, wherein the housing (30) is provided with a shutter (60) configured to carry in and out of the multi-well plate insertion unit (100).

13. The nucleic acid amplification test apparatus of claim 1, wherein the sealing means comprises a sealing film (51) or a sealing cap (52) configured to seal the inlet of the reaction tube (1).

14. The nucleic acid amplification test apparatus of claim 1, further comprising an automatic sealing unit (300) configured to press the sealing means toward the multi-well plate (40) so that the sealing means is sealed to the inlet of the reaction tube (1),
wherein the automatic sealing unit (300) comprises a pressing part (310) provided to be movable above the sealing plate insertion unit (200) to directly or indirectly press the sealing means toward the multi-well plate (40).

15. The nucleic acid amplification test apparatus of claim 14, wherein the temperature control block (500) drives and supports the reaction tubes (1) to be elevated so that the plurality of reaction tubes (1) and the sealing means are in close contact with each other.

16. The nucleic acid amplification test apparatus of claim 14, wherein the pressing part (310) comprises:
a pressing roller (311) configured to seal the sealing means to the inlet of the reaction tube (1) through horizontal movement rolling in the state of directly or indirectly pressing the sealing means at the upper side of the sealing plate insertion unit (200); and
a roller driving part (900) configured to drive the pressing roller (311).

17. The nucleic acid amplification test apparatus of claim 14, wherein the automatic sealing unit (300) further comprises a heating part (320) provided between the pressing part (310) and the sealing plate seated on the sealing plate insertion unit (200) to create high-temperature environments at an inlet-side of the reaction tube (1) of the multi-well plate (40).

18. The nucleic acid amplification test apparatus of claim 17, wherein the heating part (320) has elasticity so that pressing force through the pressing part (310) is transmitted to the sealing means.

19. The nucleic acid amplification test apparatus of claim 17, wherein the heating part (320) has through-holes (321) corresponding to the plurality of reaction tubes (1) to detect the target nucleic acid through the fluorescence detection unit (400) disposed above the sealing plate insertion unit (200) with respect to the reaction tubes (1).

20. The nucleic acid amplification test apparatus of claim 1, wherein the fluorescence detection unit (400) comprises:
a detection part (430) configured to irradiate excitation light with which the target nucleic acid reacts toward the reaction tube (1) and sense fluorescence according to the reaction of the target nucleic acid; and
a detection driving part (440) configured to drive the detection part (430) to be movable above the sealing plate insertion unit (200).

21. The nucleic acid amplification test apparatus of claim 20, wherein the detection part (430) comprises:
a light source part (410) configured to irradiate the excitation light, which the target nucleic acid reacts, toward the reaction tube (1); and
a detection sensor (420) configured to sense the fluorescence according to the reaction of the target nucleic acid through the irradiated excitation light.

22. The nucleic acid amplification test apparatus of claim 1, further comprising a scan unit (700) configured to scan an identification information code attached to the reaction tube (1) so as to acquire target nucleic acid information.

23. The nucleic acid amplification test apparatus of claim 22, wherein the scan unit (700) scans a position information code attached to the sealing plate (50) to identify whether the sealing means exists.

24. An automatic sample analysis system comprising:
an automatic purification and dispensing apparatus (10) configured to purify and dispense a target nucleic acid to dispense the target nucleic acid into a plurality of reaction tubes (1) at a dispensing position (P); and
a nucleic acid amplification test apparatus (20) configured to measure a target nucleic acid solution, which is received from the automatic purification and dispensing apparatus (10) and accommodated in the plurality of reaction tubes (1), in real-time,
wherein the nucleic acid amplification test apparatus (20) comprises:
a housing (30) having an inner space (S1); and
a multi-well plate insertion unit (100) configured to support a multi-well plate (40) provided with a plurality of reaction tubes (1) and to be installed to be movable between the dispensing position (P) and the inner space (S1).

25. The automatic sample analysis system of claim 24, wherein the multi-well plate insertion unit (100) comprises:
a support part (110) configured to support an edge of the multi-well plate (40) in which the plurality of reaction tubes (1) are provided; and
a driving part (120) configured to drive the support part (110) between a dispensing position ()) of the automatic purification and dispensing apparatus (10) and an amplification position, at which amplification of the target nucleic acid is performed, in the housing (30).

26. The automatic sample analysis system of claim 24, wherein the housing (30) is provided with a shutter (60) configured to carry in and out of the multi-well plate insertion unit (100).
